# EUROPEAN PATENT APPLICATION

(11) **EP 2 570 089 A1**
(43) Date of publication of application: **20.03.2013**
(21) Application number: 11780655.4
(22) Date of filing: 11.05.2011
(51) Int. Cl.: A61B 17/28, A61B 1/00, A61B 19/00

(54) **FORCEPS SUPPORTING DEVICE**

(30) Priority: 11.05.2010 JP 2010109102
(71) Applicant: Kyushu University, National University Corporation, Fukuoka-shi Fukuoka 812-8581 (JP)
(72) Inventor: HASHIZUME Makoto, Fukuoka-shi Fukuoka 812-8581 (JP); KENMOTSU Hajime, Fukuoka-shi Fukuoka 812-8581 (JP); YUGE Kazuo, Narashino-shi Chiba 275-0001 (JP)
(74) Representative: Nicholls, Michael John
(86) International application number: PCT/JP2011/060887
(87) International publication number: WO 2011/142398

(57) **Abstract**

A forceps support device 1 is constituted by an endoscope holding portion 20 that holds an endoscope 5, a base portion 30 that holds the endoscope holding portion 20 so as to be rotatable in a circumferential direction of the endoscope, a forceps support portion 50 that supports forceps 40 and is attached relative to the base portion 30 so as to be movable back and forth in an extension direction of the forceps, a first drive means 60 that rotates the endoscope holding portion 20 held by the base portion 30 in the circumferential direction of the endoscope, a second drive means 70 that rotates the forceps support portion 50 and the base portion 30 in the circumferential direction of the endoscope with the endoscope holding portion 20 as a fulcrum, and a third drive means 80 that moves the forceps support portion 50 back and forth in the extension direction of the forceps.

## Description

### Technical Field

The present invention relates to a forceps support device, and more particularly to a forceps support device that enables forceps to be operated with an endoscopic image in a fixed state, in endoscopic treatment and the like performed on intra-abdominal organs, the gastrointestinal tract, which is a hollow organ, and the like of the human body.

### Background Art

In recent years, in the case of treating diseases of the gastrointestinal tract, which is hollow organ, and the like of the human body, such as early stomach cancer where the tumor is confined to the mucosal lining, for example, it has become standard practice to use an endoscope to perform excision using forceps removed from a port of the endoscope, instead of performing a laparotomy or surgery using a laparoscope as was formally the case.

Furthermore, in the last few years, in order to reduce invasiveness to the body as much as possible, a new procedure (NOTES: Natural Orifice Translumenal Endoscopic Surgery) that avoids leaving an incision mark on the body by using a natural opening in the body, such as the mouth, the anus, or, in the case of women, the vagina, as the portal of entry for inserting an endoscope, making a small incision in the lumen wall of these openings to allow the endoscope to be moved into the abdominal cavity, and using diagnosis, the aforementioned forceps or the like has also begun to be performed in normal laparotomies or intra-abdominal surgery performed using a laparoscope.

An endoscopic surgical robot such as disclosed in Patent Literature 1, for example, is used in such endoscopic treatment. This endoscopic surgical robot includes an endoscope, a pair of forceps arms, a head support portion that supports the endoscope and the pair of forceps arms, and forceps portions provided at the distal ends of the forceps arms.

Each of the forceps portions in this case is constituted by a first sleeve attached to the head support portion, a second sleeve coupled to the first sleeve via a first flexible tube, a second flexible tube coupled to the second sleeve, a pair of forceps attached in an openable and closeable manner to the distal end of the second flexible tube, three oscillating levers pivotably attached around the second sleeve, wires for opening and closing the pair of forceps, and wires for pivoting the oscillating levers.

With an endoscopic surgical robot constituted such as above, by manipulating the wires to pivot one of the oscillating levers, the first flexible tube tracks that oscillating lever and bends at the first sleeve, and the pair of forceps tracks the first flexible tube and pivots in the same direction via the second sleeve and the second flexible tube. Also, by manipulating the wires to pivot another of the oscillating levers in a state in which the first flexible tube is bent, the first flexible tube tracks that oscillating lever and bends further in that direction, and the pair of forceps tracks the bending of the first flexible tube and rotates in that direction via the second sleeve and the second flexible tube.

Prescribed treatment can be performed on a disease of the gastrointestinal tract or the like, by translumenally inserting an endoscopic surgery robot having functions such as the above into the body, pivoting or rotating the pair of forceps by pivoting the oscillating levers through manipulation of the wires, positioning the pair of forceps in a position corresponding to the disease of the gastrointestinal tract or the like, and operating the wires with the pair of forceps in that position to open and close the pair of forceps.

### Citation List

### Patent Literature

Patent Document 1: JP 2004-180781A

### Disclosure of the Invention

### Problem to be Solved by the Invention

Incidentally, in the case of a conventional endoscopic surgery robot, forceps can be positioned inside the patient's body by pivoting or rotating the forceps through the manipulation of wires. Meanwhile, the physician operating the endoscopic surgery robot that has thus positioned the forceps is in a position to operate the forceps while viewing an image of the affected area or the like sent from the endoscope. However, in this case, since the arms of the forceps facing the affected area occupy a large part of the visual field of the image, it is difficult for the physician to perform dynamic operations such as so-called "turning over" and "picking up" operations, for example, that take up the entire visual field captured by the endoscope centering on the affected area, while viewing the affected area. Also, in the case of performing various operations using forceps, an IT knife or the like through the channel of an endoscope, the endoscope needs to be moved in accordance with the position of the affected area, as a result of which the visual field captured by the endoscope also changes from before the forceps operation.

For example, assume that an operation that involves turning over a tongue-shaped site formed by performing a semicircular exfoliation with an IT knife or the like with which the endoscopic surgery robot is equipped needs to be performed with forceps on a prescribed disease site. With a conventional endoscopic surgery robot, the physician initially positions the forceps and is able to perform the exfoliation operation while checking the endoscopic image. However, when the physician attempts to perform the turning over operation using the forceps, the arms of the forceps facing the affected area occupy a large part of the visual field of the image, making it difficult to view the affected area. Also, in performing the operation using the channel, the endoscope also moves due to the reaction force at the fulcrum of movement of the forceps, resulting in the captured visual field changing. Therefore, it is difficult to reliably operate the forceps while checking the endoscopic image.

The present invention was made in view of problems such as the above, and has as an object to provide a forceps support device that enables forceps to be operated with the endoscopic image in a fixed state.

### Solution to Problem

In order to solve problems such as the above, the present invention employs means such as the following.

That is, the invention according to claim 1 is a forceps support device for supporting forceps used in endoscopic treatment, comprising an endoscope holding portion that holds an endoscope, a base portion that holds the endoscope holding portion so as to be rotatable in a circumferential direction of the endoscope, a forceps support portion that supports the forceps and is attached relative to the base portion so as to be movable back and forth in an extension direction of the forceps, a first drive means for rotating the endoscope holding portion held by the base portion in the circumferential direction of the endoscope, a second drive means for rotating the forceps support portion and the base portion in the circumferential direction of the endoscope with the endoscope holding portion as a fulcrum, and a third drive means for moving the forceps support portion back and forth in the extension direction of the forceps.

According to the forceps support device of the present invention, the endoscope (i.e., which is held in the endoscope holding portion) that provides an image of an affected area or the like to a physician is held so as to be rotatable in a base portion in the circumferential direction of the endoscope. Also, the endoscope can be rotated in the circumferential direction of the endoscope by the first drive means, independently of the base portion (i.e., to which the forceps support portion is attached). Also, the forceps support portion and base portion (i.e., effectively the forceps) can be rotated in the circumferential direction of the endoscope by the second drive means, independently of the endoscope (i.e., which is held in the endoscope holding portion), with the endoscope holding portion as a fulcrum. In other words, the endoscope and the endoscope holding portion can rotate idly relative to the base portion and the like, that is, independently of the surroundings thereof, which is essentially saying that the forceps can be operated with the endoscopic image in a fixed state.

Also, the forceps support portion can be moved back and forth in the extension direction of the forceps relative to the base portion by the third drive means. As a result, the effect of being able to precisely execute operations such as positioning forceps and turning over a prescribed site, according to operations by the physician, independently of the posture of the endoscope, that is, without changing the visual field of the endoscopic image, is attained, together with the forceps support portion and the base portion (i.e., effectively the forceps) being able to rotate in the circumferential direction of the endoscope using the second drive means, independently of the endoscope.

Also, the invention according to claim 2 is the forceps support device disclosed in claim 1, wherein the forceps support portion rotatably supports the forceps, and the forceps support device comprises a fourth drive means for rotating the forceps supported by the forceps support portion.

According to the forceps support device of the present invention, the forceps themselves can be rotated, facilitating use of the forceps to respond to various situations.

Furthermore, the invention according to claim 3 is the forceps support device disclosed in claim 1 or 2, wherein the forceps support portion includes a main portion that is attached relative to the base portion and a tiltable portion that is attached to the main portion and is tiltable up and down, and the forceps support device comprises a fifth drive means for tilting the tiltable portion up and down.

According to the forceps support device of the present invention, positioning and the like can also be performed with regard to the range in which the forceps can be tilted by the tiltable portion.

Furthermore, the invention according to claim 4 is the forceps support device disclosed in claim 3, wherein the tiltable portion has a locking portion including a fitting body that fits into a profile of a proximal end of the forceps that are supported, a pair of leaf spring bodies that are integrally formed with the fitting body and biased in order to fit the fitting body into the profile of the proximal end of the forceps by obtaining a reaction force from an inner wall of the tiltable portion around the proximal end of the forceps, and a hinge body that turnably connects the leaf spring bodies to the tiltable portion, and the forceps support device comprises a sixth drive means for turning the fitting body upward and the leaf spring bodies downward with the hinge body as a fulcrum, so that a fitted state of the fitting body with the proximal end of the forceps is released.

According to the forceps support device of the present invention, in the case where the forceps need to be interchanged according to the situation, the forceps can be presented for interchange by temporarily releasing the locked state of the forceps supported by the tiltable portion, that is, the state in which the fitting body is fitted into the profile of the proximal end of the forceps. Forceps need to be interchanged according to the application, with such a configuration facilitating the interchange operation.

### Advantageous Effects of the Invention

As described above, the forceps support device of the present invention enables forceps to be operated with the endoscopic image in a fixed state.

### Brief Description of Drawings

FIG. 1 is a perspective view showing an exemplary configuration of a forceps support device in an embodiment.
FIG. 2 is an illustrative diagram showing a manner in which the forceps support device in the embodiment moves back and forth and is tiltable.
FIG. 3 is an illustrative diagram showing a manner in which the forceps support device in the embodiment performs a turning over operation.
FIG. 4 is an illustrative diagram showing a manner in which a fitted state of the forceps support device in the embodiment is released.

### Description of Embodiments

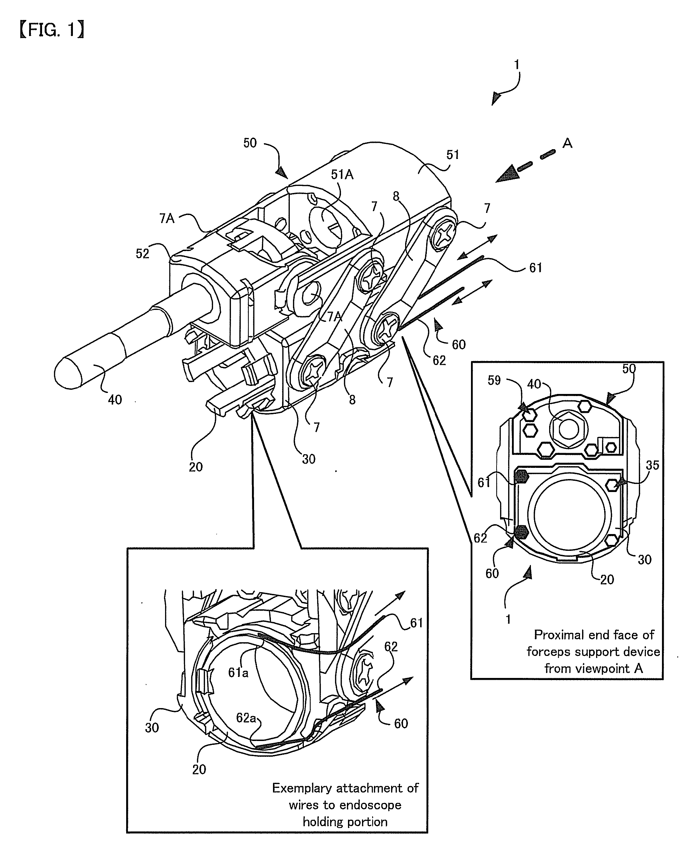

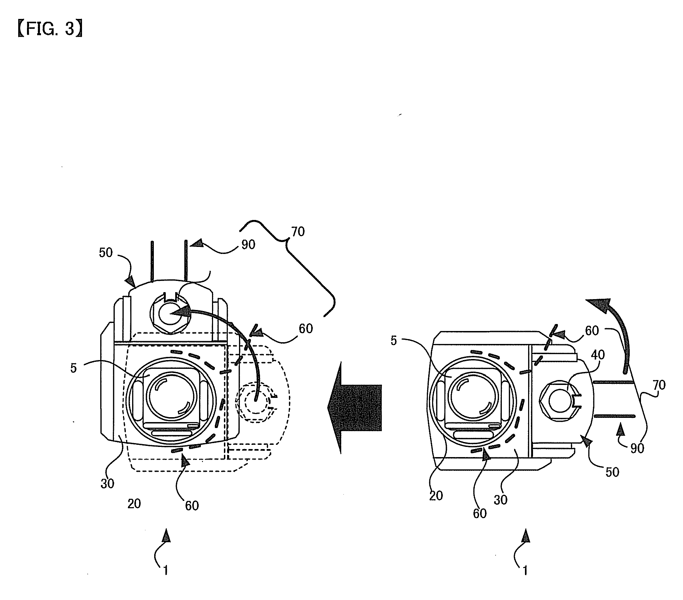

Hereafter, an embodiment of the present invention will be described with reference to the drawings. FIG. 1 is a perspective view showing an exemplary configuration of the forceps support device in the present embodiment. A forceps support device 1 in the present embodiment is effective for supporting forceps used in endoscopic treatment, in minimally invasive endoscopic treatment performed on diseases of the intra-abdominal organs or the like, for example, and, as shown in the diagram, is provided with an endoscope holding portion 20 that holds an endoscope 5 (see FIG. 3), a base portion 30 that holds the endoscope holding portion 20 so as to be rotatable in a circumferential direction of the endoscope, and a forceps support portion 50 that supports forceps 40 and is attached relative to the base portion 30 so as to be movable back and forth in an extension direction of the forceps relative to the base portion 30. Note that, in the present embodiment, the shape of the endoscope holding portion 20 is defined so that the endoscope holding portion 20 is able to support existing endoscopes without their modification.

Also, the forceps support device 1 is provided with a first drive means 60 for rotating the endoscope holding portion 20 held by the base portion 30 in the circumferential direction of the endoscope, a second drive means 70 (see FIG. 3) for rotating the forceps support portion 50 and the base portion 30 in the circumferential direction of the endoscope with the endoscope holding portion 20 as a fulcrum, and a third drive means 80 for moving the forceps support portion 50 back and forth in the extension direction of the forceps.

As for the first drive means 60, one end is coupled to various gripping mechanisms (not shown) such as levers or the like that are handy to the operator (physician, etc.) of the forceps support device 1, and the other end is coupled to a prescribed site of the endoscope holding portion 20 of the forceps support device 1 which is to be driven, with wires 61 and 62 that can advance and retreat in the longitudinal direction thereof being applicable. The first drive means 60 is assumed to also be provided with a first actuator (not shown) that drives these wires 61 and 62. Respective ends 61a and 62a of the wires 61 and 62 are, for example, attached to opposing positions on the outer peripheral diameter of the endoscope holding portion 20. Also, respective other ends 61b and 62b of the wires 61 and 62 are guided by wire channels 35 provided in the casing of the base portion 30, as shown in the diagram, to the actuator and gripping mechanisms.

By operating the first actuator of the first drive means 60 to, for example, draw in the wire 61 toward the operator (e.g., assuming the wire 62 is unrestrained at this time), the endoscope holding portion 20 rotates clockwise in the circumferential direction of the endoscope, within the inner space of the base portion 30. The amount of rotation will naturally depend on the amount by which the wire 61 is drawn in. On the other hand, by operating the first actuator of the first drive means 60 to, for example, draw in the wire 62 toward the operator (e.g., assuming the wire 61 is unrestrained at this time), the endoscope holding portion 20 rotates counterclockwise in the circumferential direction of the endoscope, within the inner space of the base portion 30. Also, the inner space of the base portion 30 and the outer peripheral surface of the endoscope holding portion 20 are both smooth enough for such rotation to be executed smoothly, or an appropriate slide mechanism such as a roller or ball bearings is provided in at least one of the inner space the base portion 30 and the outer peripheral surface of the endoscope holding portion 20.

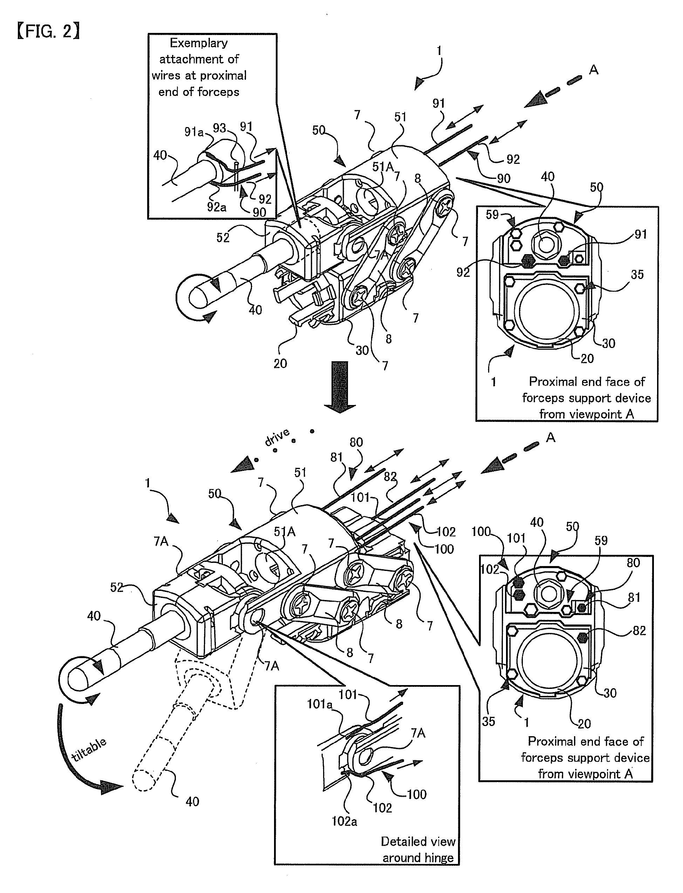

FIG. 2 is an illustrative diagram showing a manner in which the forceps support device in the present embodiment moves back and forth and is tiltable. Also, as for the third drive means 80, one end is coupled to various gripping mechanisms (not shown) such as levers or the like that are handy to the operator (physician, etc.) of the forceps support device 1, and the other end is coupled to a prescribed site of the forceps support portion 50 of the forceps support device 1 which is to be driven, with wires 81 and 82 that can advance and retreat in the longitudinal direction thereof being applicable. The third drive means 80 is assumed to also be provided with a third actuator (not shown) that drives these wires 81 and 82. One end of the wire 81 is attached to the proximal end of the forceps support portion 500, and one end of the wire 82 is attached to the proximal end of the base portion 30. Also, the other end of the wire 81 is guided by a wire channel 55 provided in the casing of the forceps support portion 50 and the other end of the wire 82 is guided by a wire channel 35 provided in the casing of the base portion 30, as shown in the diagram, to the actuator and gripping mechanisms.

By operating the third actuator of the third drive means 80 to, for example, feed out the wire 81 in the extension direction of the forceps 40 away from the operator (e.g., the wire 82 is assumed to be fixed at this time), the forceps support portion 50 slides in the extension direction of the forceps 40, that is, moves forward, from the base portion 30. The amount of movement will naturally depend on the amount by which the wire 81 is fed out. On the other hand, by operating the third actuator of the third drive means 80 to, for example, draw in the wire 81 toward the operator (e.g., the wire 82 is assumed to be fixed at this time), the forceps support portion 50 slides toward the operator. Also, the base portion 30 and the forceps support portion 50 are assumed to be coupled by arms 8 of prescribed length with hinges 7 as fulcrums, so that such movement can be executed smoothly. The range of movement of the hinges 7 and the length of the arms 8 are set beforehand depending on how far the forceps 40 will be moved back and forth (existing techniques may be used for investigating and setting these variables). The range over which the forceps 40 can move back and forth from the base portion 30 naturally increases as the range of movement of the hinges 7 increases, that is, as the range of rotation increases, and as the length of the arms 8 increases.

Also, in the forceps support device 1, the forceps support portion 50 preferably supports the forceps 40 so as to be rotatable. In this case, the forceps support device 1 is provided with a fourth drive means 90 for rotating the forceps 40 supported by the forceps support portion 50. As for the fourth drive means 90, one end is coupled to various gripping mechanisms (not shown) such as levers or the like that are handy to the operator (physician, etc.) of the forceps support device 1, and the other end is coupled to a prescribed site of the forceps 40 which is to be driven, with wires 91 and 92 that can be advanced and retracted in the longitudinal direction thereof through an appropriate mechanism 93 such as a pulley or a roller being applicable. The fourth drive means 90 is assumed to also be provided with a fourth actuator (not shown) that drives these wires 91 and 92. Respective ends 91a and 92a of the wires 91 and 92 are, for example, attached to opposing positions on the outer peripheral diameter of the forceps 40. Also, the other ends of the wires 91 and 92 are guided by wire channels 59 provided in the casing of the forceps support portion 50, as shown in the diagram, to the actuator and gripping mechanisms.

By operating the fourth actuator of the fourth drive means 90 to, for example, draw in the wire 91 toward the operator (e.g., assuming the wire 92 is unrestrained at this time), the forceps 40 rotates, that is, axially rotates, clockwise about its axis in the extension direction (direction of arrow at the distal end of the forceps in FIG. 2). The amount of rotation will naturally depend to amount by which the wire 91 is drawn in. On the other hand, by operating the fourth actuator of the fourth drive means 90 to, for example, draw in the wire 92 toward the operator (e.g., assuming the wire 91 is unrestrained at this time), the forceps 40 rotates counterclockwise about its axis in the extension direction. Also, the forceps holding site of the forceps support portion 50 and the outer peripheral surface of the forceps 40 are both smooth enough for such rotation to be executed smoothly, or an appropriate slide mechanism such as a roller or ball bearings is provided in at least one of the forceps holding site of the forceps support portion 50 and the outer peripheral surface of the forceps 40.

Also, in the forceps support device 1, the forceps support portion 50 preferably is constituted by a main portion 51 that is attached relative to the base portion 30, and a tiltable portion 52 that is attached to the main portion 51 and is tiltable up and down. In the example shown in FIG. 2, the main portion 51 is the portion coupled to the base portion 30 by the arms 8 of prescribed length with the hinges 7 as fulcrums, whereas the tiltable portion 52 is the portion turnably coupled to the main portion 51 via a hinge 7A.

In this case, the forceps support device 1 is provided with a fifth drive means 100 for tilting the tiltable portion 52 up and down. As for the fifth drive means 100, one end is coupled to various gripping mechanisms (not shown) such as levers or the like that are handy to the operator (physician, etc.) of the forceps support device 1, and the other end is coupled to a prescribed site of the tiltable portion 52 (i.e., the forceps 40) which is to be driven, with wires 101 and 102 that can advance and retreat in the longitudinal direction thereof being applicable. The fifth drive means 100 is assumed to also be provided with a fifth actuator (not shown) that drives these wires 101 and 102. Respective ends 101a and 102a of the wires 101 and 102 are, for example, attached to opposing positions of the tiltable portion 52 across the hinge 7A. Also, the other ends of the wires 101 and 102 are, for example, guided by wire channels 59 provided in the casing of the forceps support portion 50, as shown in the diagram, to the actuator and gripping mechanisms.

By operating the fifth actuator of the fifth drive means 100 to, for example, draw in the wire 102 toward the operator (e.g., assuming the wire 101 is unrestrained at this time), the tiltable portion 52 drops downward with the hinge 7A as a fulcrum, so as to be oriented with the distal end of the forceps facing downward. This tiltable amount will naturally depend on the amount by which the wire 102 is drawn in. On the other hand, by operating the fifth actuator of the fifth drive means 100 to, for example, draw in the wire 101 toward the operator (e.g., assuming the wire 102 is unrestrained at this time), the tiltable portion 52 swings upward with the hinge 7A as a fulcrum, so as be oriented with the distal end of the forceps facing upward.

Also, the range of movement of the hinge 7A is set beforehand depending on the amount by which the tiltable portion 52, that is, the forceps 40 will move up and down (existing techniques may be used for investigating and setting this variable). The range over which the forceps 40 can move up and down naturally increases as the range of movement of the hinge 7A increases, that is, as the range of rotation, increases.

Note that the second drive means 70 of the abovementioned drive means can be realized by combining the first drive means 60 and the fourth drive means 90. This second drive means 70 serves as a drive means for rotating the forceps support portion 50 and the base portion 30 in the circumferential direction of the endoscope with the endoscope holding portion 20 as a fulcrum. In this case, as shown in FIG. 3, if an operation for rotating the fourth drive means 90 is performed with the first drive means 60 fixed, the endoscope holding portion 20, that is, the base portion 30 (and the forceps support portion 50 coupled thereto), which rotatably contacts the endoscope 5, will rotate about the endoscope 5, whose movement is regulated by the first drive means 60. In order to realize such an operation, in addition to the functions of drawing in and feeding out wires such as mentioned above, the fourth drive means 90 is provided with a function of conveying a wire twisting operation of the operator or the fourth actuator to the distal ends of the wires, for example. This rotation operation also enables the affected area to be turned over by rotating the forceps 40 after taking hold of the tongue-like affected area with the forceps 40.

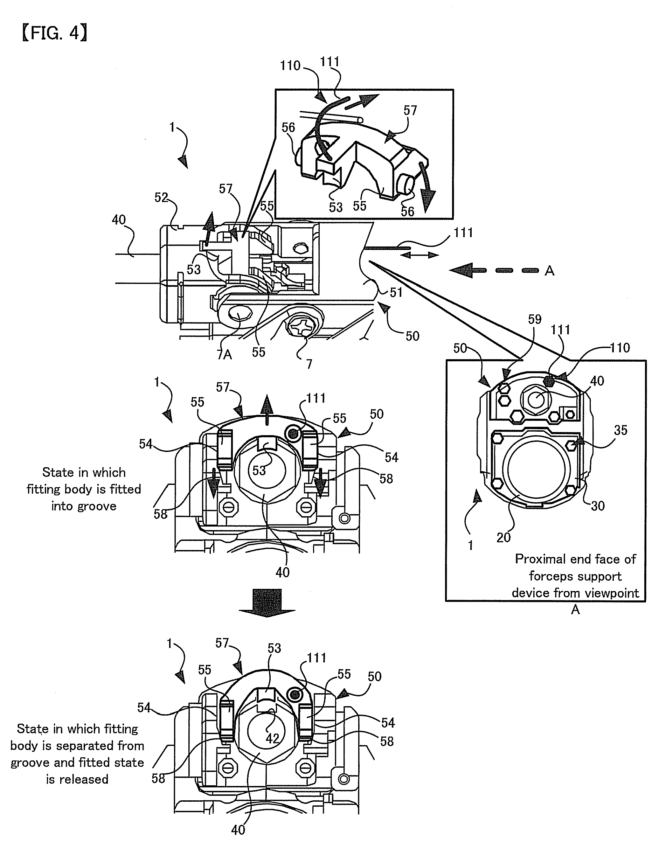

Note that the forceps support device 1 of the present embodiment is provided with a mechanism that enables the forceps 40 to be interchanged. FIG. 4 is an illustrative diagram showing a manner in which the fixed state of the forceps support device in the present embodiment is released. In this case, the tiltable portion 52 of the forceps support portion 50 is provided with a locking portion 57 including a fitting body 53 that fits the profile of the proximal end of the supported forceps, a pair of leaf spring bodies 55 that are integrally formed with the fitting body 53 and biased in order to fit the fitting body 53 into the profile of the proximal end of the forceps by obtaining a reaction force from an inner wall 54 of the tiltable portion around the proximal end of the forceps, and a hinge body 56 that turnably connects the leaf spring bodies 55 to the tiltable portion 52.

The proximal end of the forceps 40 is assumed to have a hex nut shape, for example, and a portion thereof is provided with a groove 42 (i.e., profile). The fitting body 53 is the portion that is provided with a profile that conforms to the shape of the inner space of the groove 42 and fits therein at this time. Also, a given area of the inner wall 54 of the tiltable portion around the proximal end of the forceps 40 is a vertical wall, and the area from there down forms a sloping wall 58 that narrows downwardly (toward the base portion 30) in the shape of an earthenware mortar, for example. On the other hand, the pair of leaf spring bodies 55 are positioned abutting the vertical wall portion of the inner wall 54 of the tiltable portion with a prescribed pressure when the forceps 40 have been set (i.e., when the fitting body 53 has been fitted into the groove 42). The pair of leaf spring bodies 55 can obtain a reaction force from the vertical wall portion, due to the diameter of the pair of leaf spring bodies 55 being appropriately larger than the diameter of the inner space of the vertical wall portion of the inner wall 54 of the tiltable portion.

In this case, the forceps support device 1 is provided with a sixth drive means 110 for turning the fitting body 53 upward and the leaf spring bodies 55 downward with the hinge body 56 as a fulcrum, such that the fitted state of the fitting body 53 with the proximal end of the forceps is released.

As for the sixth drive means 110, one end is coupled to various gripping mechanisms (not shown), such as levers or the like that are handy to the operator (physician, etc.) of the forceps support device 1, and the other end is coupled to a prescribed site (e.g., the fitting body 53) of the tiltable portion 52 which is to be driven, with a wire 111 that can advance and retreat in the longitudinal direction thereof being applicable. The sixth drive means 110 is assumed to also be provided with a sixth actuator (not shown) that drives this wire 111. An end 111a of the wire 111 is attached near the distal end of the fitting body 53 of the tiltable portion 52, for example. Also, the other end of the wire 111 is guided by a wire channel 59 provided in the casing of the forceps support portion 50, as shown in the diagram, to the actuator and gripping mechanisms.

By operating the sixth actuator of the sixth drive means 110 to, for example, draw in the wire 111 toward the operator by a prescribed amount, the fitting body 53 will spring upward with the hinge body 56 as a fulcrum, and separate from the groove 42. On the other hand, the leaf spring bodies 55 sink downward with the hinge body 56 as a fulcrum. At this time, the leaf spring bodies 55 will be pushed against the area of the inner wall 54 of the tiltable portion below the vertical wall (toward the base portion 30), that is, against the earthenware mortar-shaped sloping wall 58, while bending. In such a state, the fitting body 53 will be separated from the groove 42 of the forceps 40, enabling the forceps 40 to be interchanged.

In this case, a situation can be envisioned where, for example, the main portion 51 of the forceps support portion 50 is provided with a mechanism for delivering the forceps 40 (roller that grips and moves the forceps 40 back and forth inside an interchanging hole 51A and a drive device therefor, etc.) and interchangeable forceps. The operator operates the sixth actuator of the sixth drive means 110 to separate the fitting body 53 from the groove 42, and operates the delivery mechanism to collect the forceps 40 in the inner space of the main portion 51 via the interchanging hole 51A, while similarly operating the delivery mechanism to send the interchangeable forceps to the forceps support position of the tiltable portion 52 via the interchanging hole 51A.

On the other hand, by operating the sixth actuator of the sixth drive means 110 to, for example, feed out the wire 111 in the extension direction of the forceps away from the operator, the fitting body 53 will sink downward with the hinge body 56 as a fulcrum and fit into the groove 42 of the interchangeable forceps. On the other hand, the leaf spring bodies 55 that are also being pushed against the sloping wall 58 while bending are released from this pushing force and spring upward with the hinge body 56 as a fulcrum. At this time, the leaf spring bodies 55 will be released from their bent state and return to the area of the vertical wall of the inner wall 54 of the tiltable portion. In other words, the fitting body 53 again fits into the groove 42 of the forceps 40, and the forceps 40 are fixed in place.

Note that the abovementioned wires may of course be operated manually without using drive means, the wires may be replaced by rods, and these wires or rods can, furthermore, also be driven by other drive means instead of the above drive means.

According to the present embodiment, by having an axis of operation centered on the endoscope (image), the arms of the forceps hardly appear in the image of the affected area, even when dynamic operations such as "turned over" and "picking up" are performed, allowing an uninterrupted view of the affected area. Therefore, the affect of dynamic operations on the image of the affected area can be reduced as much as possible, enabling safer procedures to be dynamically performed.

### Reference Signs List

- 1: forceps support device
- 5: endoscope
- 7, 7A: hinges
- 8: arm
- 20: endoscope holding portion
- 30: base portion
- 35: wire channel
- 40: forceps
- 42: groove
- 50: forceps support portion
- 51: main portion
- 52: tiltable portion
- 53: fitting body
- 54: inner wall of tiltable portion
- 55: leaf spring body
- 56: hinge body
- 57: locking portion
- 58: sloping wall
- 59: wire channel
- 60: first drive mean
- 61, 62: wires
- 70: second drive means
- 80: third drive means
- 81, 82: wires
- 90: fourth drive means
- 91, 91: wires
- 100: fifth drive means
- 101, 102: wires
- 110: sixth drive means
- 111: wire

## Claims

1. A forceps support device for supporting forceps used in endoscopic treatment, comprising:
an endoscope holding portion that holds an endoscope;
a base portion that holds the endoscope holding portion so as to be rotatable in a circumferential direction of the endoscope;
a forceps support portion that supports the forceps and is attached relative to the base portion so as to be movable back and forth in an extension direction of the forceps;
a first drive means for rotating the endoscope holding portion held by the base portion in the circumferential direction of the endoscope;
a second drive means for rotating the forceps support portion and the base portion in the circumferential direction of the endoscope with the endoscope holding portion as a fulcrum; and
a third drive means for moving the forceps support portion back and forth in the extension direction of the forceps.

2. The forceps support device according to claim 1,
wherein the forceps support portion rotatably supports the forceps, and
the forceps support device comprises a fourth drive means for rotating the forceps supported by the forceps support portion.

3. The forceps support device according to claim 1 or 2,
wherein the forceps support portion includes a main portion that is attached relative to the base portion and a tiltable portion that is attached to the main portion and is tiltable up and down, and
the forceps support device comprises a fifth drive means for tilting the tiltable portion up and down.

4. The forceps support device according to claim 3,
wherein the tiltable portion has a locking portion including a fitting body that fits into a profile of a proximal end of the forceps that are supported, a pair of leaf spring bodies that are integrally formed with the fitting body and biased in order to fit the fitting body into the profile of the proximal end of the forceps by obtaining a reaction force from an inner wall of the tiltable portion around the proximal end of the forceps, and a hinge body that turnably connects the leaf spring bodies to the tiltable portion, and
the forceps support device comprises a sixth drive means for turning the fitting body upward and the leaf spring bodies downward with the hinge body as a fulcrum, so that a fitted state of the fitting body with the proximal end of the forceps is released.
